# EUROPEAN PATENT APPLICATION

(11) **EP 4 825 836 A1**
(43) Date of publication of application: **21.10.2026**
(21) Application number: 24906579.8
(22) Date of filing: 21.12.2024
(51) Int. Cl.: C07C 17/278, C07C 19/10, C07C 21/18

(54) **METHODS FOR PREPARING FLUORINE-CONTAINING PROPYLENE AND INTERMEDIATE THEREOF**

(30) Priority: 22.12.2023 CN 202311780931
(71) Applicant: Zhejiang Lantian Environmental Protection Hi-Tech Co., Ltd., Hangzhou, Zhejiang 310018 (CN); Sinochem Lantian Co., Ltd., Hangzhou, Zhejiang 310051 (CN)
(72) Inventor: LIU, Wucan, Hangzhou, Zhejiang 310051 (CN); LIU, Minyang, Hangzhou, Zhejiang 310051 (CN); YU, Houlin, Hangzhou, Zhejiang 310051 (CN); YU, Wanjin, Hangzhou, Zhejiang 310051 (CN); ZHANG, Jianjun, Hangzhou, Zhejiang 310051 (CN)
(74) Representative: Meyer-Dulheuer MD Legal Patentanwälte PartG mbB
(86) International application number: PCT/CN2024/141247
(87) International publication number: WO 2025/131109

(57) **Abstract**

Methods for preparing fluorine-containing propylene and a fluorochloropropane intermediate thereof are provided. The method for preparing the fluorochloropropane intermediate includes: subjecting dichloromethane, difluoromethane, and a fluorine-containing olefin of general formula CF₂=CR₁R₂ to a liquid-phase addition reaction under the action of an addition catalyst to prepare fluorochloropropane of general formula of CF₃CR₁R₂CH₂Cl, wherein the content of the fluorine-containing olefin in a reaction system is monitored during the reaction process, and the content of the fluorine-containing olefin thereof is controlled to be less than or equal to 5%; and the addition catalyst is a mixed catalyst including a metal fluoride, a metal chloride and a metal oxide, and the metal is selected from metal elements in group IIIA, IVB, or VA. The obtained fluorochloropropane intermediate is subjected to a dehydrochlorination reaction under the action of activated carbon or a metal alkali solution to obtain fluorine-containing propylene. The method of the present invention has the advantages of a simple process flow, mild reaction conditions, easy product separation, good product purity, a remarkably improved service life of the addition catalyst, etc.

## Description

### TECHNICAL FIELD

The present invention relates to preparation of fluorochloropropane and a fluorine-containing propylene, in particular, to a method for preparing a fluorine-containing propylene, which includes preparing a fluorochloropropane intermediate having a general formula of CF₃CR₁R₂CH₂Cl from a fluorine-containing olefin having a general formula of CF₂=CR₁R₂, dichloromethane, difluoromethane under the action of a metal LEWIS acid, and subjecting the fluorochloropropane intermediate to a dehydrochlorination reaction to obtain the fluorine-containing propylene.

### BACKGROUND

3,3,3-Trifluoropropene, 2-chloro-3,3,3-trifluoropropene, 2,3,3,3-tetrafluoropropene, and 1-chloro-2,3,3,3-tetrafluoropropene are four common kinds of fluorine-containing propylene. In particular, 2,3,3,3-tetrafluoropropene, owing to its superior environmental performance and refrigeration performance similar to that of 1,1,1,2-tetrafluoroethane (R134a), has gradually replaced R134a as a mainstream refrigerant in the air conditioning field of new energy vehicles. Preparation of fluorine-containing propylene is usually achieved by dehydrochlorination reaction of fluorochloropropane; therefore, how to obtain fluorochloropropane is key to the preparation of fluorine-containing propylene.

1-Chloro-3,3,3-trifluoropropane (HCFC-253fb), 2,3-dichloro-1,1,1-trifluoropropane (HCFC-243db), 3-chloro-1,1,1,2-tetrafluoropropane (HCFC-244eb), and 2,3-dichloro-1,1,1,2-tetrafluoropropane (HCFC-234ba) are important fluorine-containing fine chemicals. They can be used to prepare novel refrigerants such as HCFO-1224yd and HFO-1234yf by dehalogenation reactions, and thus have great application potential.

Currently, there are relatively few studies on the synthesis of 1-chloro-3,3,3-trifluoropropane, 3-chloro-1,1,1,2-tetrafluoropropane, and 2,3-dichloro-1,1,1,2-tetrafluoropropane compounds.

### (I) Research on synthesis of 1-chloro-3,3,3-trifluoropropane

US Patent US2644845A discloses a method for obtaining 1-chloro-3,3,3-trifluoropropane by photo-chlorination using 1,1,1-trifluoropropane and chlorine as raw materials. The method has a simple process, but the product yield is low. Under irradiation with a 200 W incandescent lamp and a reaction temperature of 110 degrees centigrade, the yield of 1-chloro-3,3,3-trifluoropropane is only 56%.

China patent CN1488614A discloses a two-step method for synthesizing 1-chloro-3,3,3-trifluoropropane, including: 1) preparing an intermediate 1,3,3,3-tetrachloropropane from carbon tetrachloride and ethylene under the action of an initiator such as ferric chloride or phosphate esters; and 2) subjecting the 1,3,3,3-tetrachloropropane to a liquid-phase fluorination reaction with anhydrous hydrogen fluoride under the action of a catalyst such as tin tetrachloride or titanium tetrachloride to produce 1-chloro-3,3,3-trifluoropropane. This process route has low requirements for reaction equipment, low energy consumption, and easy separation and purification of the product. However, it suffers from low raw material utilization. Under optimal conditions, the conversion rate of carbon tetrachloride is 75%, and the product yield in the fluorination step is only 65%.

China patent CN107162871A discloses a two-step method for preparing 1-chloro-3,3,3-trifluoropropane, using activated carbon as a catalyst and trifluoromethane as a starting material. A catalytic chlorination reaction is carried out in a fixed-bed reactor to obtain the intermediate trifluorochloromethane, which is then subjected to a telomerization reaction with ethylene under the action of an iron-molybdenum catalyst to generate 1-chloro-3,3,3-trifluoropropane. The yield of this process can reach 86%, but the intermediate product requires purification steps such as alkali washing, drying, and extraction, making the post-treatment complex. In addition, the reaction temperature is high, with the gas-phase chlorination temperature exceeding 300 degrees centigrade.

### (II) Research on synthesis of 2,3-dichloro-1,1,1-trifluoropropane

China patent CN101168494A discloses a method for synthesizing 2,3-dichloro-1,1,1-trifluoropropane by liquid-phase fluorination using 1,1,1,2,3-pentachloropropane as a raw material under the action of catalysts such as chromium fluoride, magnesium fluoride, and titanium fluoride. The process route is simple and the raw materials are readily available, but the product yield is low. After reacting at a temperature in a range of 90 degrees centigrade to 130 degrees centigrade for 4 hours to 10 hours, the product yield is only about 85%.

China patent CN10197936A discloses a method for synthesizing 2,3-dichloro-1,1,1-trifluoropropane in a tubular reactor or a tank reactor using 1,1,1-trifluoropropene and chlorine as raw materials under irradiation with an ultraviolet lamp. This method achieves a raw material conversion rate of 95% and a selectivity exceeding 90%, but it has high equipment requirements and poses significant hazards to human health.

### (III) Research on synthesis of 3-chloro-1,1,1,2-tetrafluoropropane

Patent WO2019003896A discloses a method for preparing 2-chloro-1,1,1,2-tetrafluoropropane and/or 3-chloro-1,1,1,2-tetrafluoropropane by liquid-phase photocatalytic chlorination using 1,1,1,2-tetrafluoropropane as a raw material. The process is simple, but the main product is 2-chloro-1,1,1,2-tetrafluoropropane, with selectivity for 3-chloro-1,1,1,2-tetrafluoropropane below 20%. Moreover, there are many by-products with similar boiling points, making separation and purification difficult.

Patent CN115023490A discloses a method for preparing 3-chloro-1,1,1,2-tetrafluoropropane, in which 2,3,3,3-tetrafluoropropene is hydrogenated under the action of a palladium-on-carbon catalyst to obtain 1,1,1,2-tetrafluoropropane, and then the 1,1,1,2-tetrafluoropropane reacts with chlorine to obtain a mixture of HCFC-244bb, HCFC-244eb, and HCFC-234ea. The mixture is then purified by distillation to obtain 3-chloro-1,1,1,2-tetrafluoropropane. However, patent CN115023490A does not disclose the reaction conditions or the reaction effect.

### (IV) Research on synthesis of 2,3-dichloro-1,1,1,2-tetrafluoropropane

Patents CN103524293A, CN105753638A, CN105777484A, etc., disclose methods for synthesizing 2,3-dichloro-1,1,1,2-tetrafluoropropane by two-step fluorination (gas-phase and liquid-phase) using a compound having a general formula of CF*₃₋ₓ*Cl*ₓ*CH*_{2-y}*Cl*_{y}*CH*_{3-z}*Cl*_{z}* as a starting material under the action of a fluorination catalyst. Under optimal conditions, the raw material conversion rate reaches 93.7% and the selectivity reaches 83.5%. However, this method requires relatively high reaction temperatures: the gas-phase fluorination temperature is in a range of 200 degrees centigrade to 350 degrees centigrade, and the liquid-phase fluorination temperature is in a range of 110 degrees centigrade to 150 degrees centigrade. Moreover, the reaction process requires a combination of gas-phase fluorination and liquid-phase fluorination, making the process difficult. Additionally, the patent does not disclose the service life of the catalyst.

In summary, in the related art, the synthesis of fluorochloropropane is either obtained by chlorination or fluorination with low product yield; or obtained by two-step reactions, but the technical route is relatively complex, the target product yield is not high, the reaction conditions are harsh, and problems such as high difficulty in product separation and purification exist.

### SUMMARY

In order to solve the technical problems above, the present invention provides a method for preparing a fluorochloropropane intermediate by an addition reaction. The method has simple process and mild reaction conditions. The product is easy to separate and has good purity. Moreover, a service life of the catalyst is significantly improved.

The object of the present invention can be achieved by following technical solutions.

A method for preparing fluorochloropropane by an addition reaction is disclosed in the present invention. The method includes following steps: subjecting dichloromethane, difluoromethane, and a fluorine-containing olefin of Formula I to a liquid-phase addition reaction under the action of an addition catalyst, so as to prepare fluorochloropropane of Formula II, and a reaction equation is as follows: 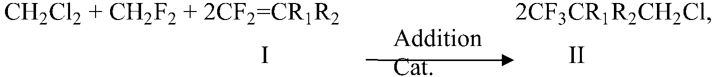 wherein R₁ is selected from hydrogen or fluorine, R₂ is selected from hydrogen or chlorine; and the addition catalyst is a mixed catalyst including a metal fluoride, a metal chloride and a metal oxide, and a metal element of the metal fluoride is selected from metal elements in group IIIA, group IVB or group VA, a metal element of the metal chloride is selected from metal elements in group IIIA, group IVB or group VA, and a metal element of the metal oxide is selected from metal elements in group IIIA, group IVB or group VA. In some embodiments, the metal is selected from the group consisting of Al, Sb, Ti, Zr, Hf, Nb, Ta, Ga, In, and any combination thereof.

In some embodiments, the metal fluoride is selected from the group consisting of AlF₃, TiF₄, SbF₅, ZrF₄, HfF₄, NbF₅, TaF₅, GaF₃, InF₃, and any combination thereof; the metal chloride is selected from the group consisting of AlCl₃, TiCl₄, SbCl₅, ZrCl₄, HfCl₄, NbCl₅, TaCl₅, GaCl₃, InCl₃, and any combination thereof; and the metal oxide is selected from the group consisting of Al₂O₃, TiO₂, Sb₂O₅, ZrO₂, HfO₂, Nb₂O₅, TaO₂, Ga₂O₃, In₂O₃, and any combination thereof.

In some embodiments, the metal fluoride is selected from the group consisting of AlF₃, TiF₄, SbF₅, ZrF₄, HfF₄, and any combination thereof; the metal chloride is selected from the group consisting of AlCl₃, TiCl₄, SbCl₅, ZrCl₄, HfCl₄, and any combination thereof; and the metal oxide is selected from the group consisting of Al₂O₃, TiO₂, Sb₂O₅, ZrO₂, HfO₂, and any combination thereof.

In some embodiments, the metal element of the metal fluoride, the metal element of the metal chloride and the metal element of the metal oxide are the same.

In some embodiments, the fluorine element, the chloride element and the oxide element in the addition catalyst sources from the corresponding metal fluoride, metal chloride and metal oxide. By adjusting a content of the metal fluoride, a content of the metal chloride and a content of the metal oxide in the addition catalyst, a content of the fluorine element, a content of the chloride element and a content of the oxide element can be adjusted.

In order to improve reaction effect, in the addition catalyst, a molar content of fluorine element is in a range of 40% to 70% of a total anion molar content, a molar content of chlorine element is in a range of 20% to 50% of the total anion molar content, and a molar content of oxygen element is in a range of 10% to 30% of the total anion molar content. In some embodiments, the molar content of fluorine element is in a range of 50% to 60% of the total anion molar content, the molar content of chlorine element is in a range of 30% to 40% of the total anion molar content, and the molar content of oxygen element is in a range of 10% to 20% of the total anion molar content.

In the present invention, the addition catalyst can efficiently dissociate the raw materials difluoromethane and dichloromethane to generate active ions such as F⁻, CH₂F⁺, Cl⁻, and CH₂Cl⁺. The addition reaction between the halogenated hydrocarbon and the halogenated olefin is electrophilic addition reaction. According to Markovnikov's rule and the electronegativity of charged groups, F⁻ and CH₂Cl⁺ can undergo a directed addition reaction with the fluorine-containing olefin compound to obtain the addition product CF₃CR₁R₂CH₂Cl with high selectivity.

The fluorine-containing olefin of the present invention is selected from the group consisting of vinylidene fluoride (CF₂=CH₂F), chlorodifluoroethylene (CF₂=CHCl), trifluoroethylene (CF₂=CHF), chlorotrifluoroethylene (CF₂=CFCl), and any combination thereof, and the corresponding fluorochloropropane are 1-chloro-3,3,3-trifluoropropane (CF₃CH₂CH₂Cl), 2,3-dichloro-1,1,1-trifluoropropane (CF₃CHClCH₂Cl), 3-chloro-1,1,1,2-tetrafluoropropane (CF₃CHFCH₂Cl), and 2,3-dichloro-1,1,1,2-tetrafluoropropane (CF₃CFClCH₂Cl), respectively.

In the method for preparing fluorochloropropane of the present invention, a molar ratio of raw materials, a molar ratio of raw materials to the addition catalyst, the reaction temperature, the reaction duration, the feeding method, etc., all affect the reaction results, and in particular, have a significant impact on the service time of the catalyst and the selectivity of the target product.

A molar ratio of the dichloromethane to the fluorine-containing olefin is in a range of 0.5 : 1 to 2 : 1. In some embodiments, the molar ratio of the dichloromethane to the fluorine-containing olefin is in a range of 0.5 : 1 to 1 : 1. In some embodiments, the molar ratio of the dichloromethane to the fluorine-containing olefin is in a range of 0.6 : 1 to 0.7 : 1.

Furthermore, a weight ratio of the dichloromethane to the addition catalyst is greater than or equal to 2. When the weight ratio of the dichloromethane to the addition catalyst is less than 2, liquid holdup in a reaction kettle is insufficient, and the reaction system cannot be effectively stirred, resulting in a decrease in product yield. In some embodiments, a weight ratio of the dichloromethane to the addition catalyst is in a range of 2 to 10. In some embodiments, the weight ratio of the dichloromethane to the addition catalyst is in a range of 4 to 6.

It was concluded from the research of the present invention that during the process for preparing fluorochloropropane by the addition reaction, the raw material fluorine-containing olefin tends to be adsorbed onto active sites of the catalyst and polymerize, thereby preventing contact between the reactants and the active sites, causing reduced catalyst activity or even catalyst deactivation. Thus, in the present invention, a content of the fluorine-containing olefin in a reaction system is monitored during a reaction process, and the content of the fluorine-containing olefin is controlled to be equal to or less than 5%. In some embodiments, the content of the fluorine-containing olefin is controlled to be equal to or less than 3%. In some embodiments, the content of the fluorine-containing olefin is controlled to be equal to or less than 1%. When the content of the fluorine-containing olefin is greater than 5%, the active sites of the catalyst will be occupied by the fluorine-containing olefin polymers, gradually resulting in catalyst deactivation.

In order to maximize the inhibition of polymerization of the fluorine-containing olefin on active sites during the reaction and further improve the service life of the catalyst, the fluorine-containing olefin of the invention is continuously fed under pressure via a metering pump, wherein the continuous feeding rate is mainly determined by a consumption rate of the fluorine-containing olefin in the reaction system. In order to prevent the fluorine-containing olefin from accumulating in the reaction system, a feeding rate of the fluorine-containing olefin is in a range of 20 g/h to 120 g/h; and in some embodiments, the feeding rate of the fluorine-containing olefin is in a range of 60 g/h to 80 g/h. When the content of the fluorine-containing olefin is monitored to be greater than 5%, the feeding rate of the fluorine-containing olefin is reduced. In actual operation, when the content of the fluorine-containing olefin is close to 5% or up to 5%, the feeding rate of the fluorine-containing olefin is moderately reduced.

Furthermore, in order to avoid catalyst deactivation caused by polymerization of the fluorine-containing olefin at the active sites of the catalyst, the fluorine-containing olefin should react completely as much as possible. In the addition reaction, a total amount of the fluorine-containing olefin satisfies a following relationship: ${n}_{{CF}_{2} = {CR}_{1} {R}_{2}} \leq \frac{1}{2} {n}_{{CH}_{2} {Cl}_{2}} , and {n}_{{CF}_{2} = {CR}_{1} {R}_{2}} \leq \frac{1}{2} {n}_{{CH}_{2} {F}_{2}} ,$ wherein *n*_{*CF*₂=*CR*₁*R*₂} represents a molar amount of the fluorine-containing olefin, *n*_{*CH*₂*F*₂} represents a molar amount of the difluoromethane, and *n*_{*CH*₂*Cl*₂} represents a molar amount of the dichloromethane. In actual reaction, the fluorine-containing olefin is continuously fed. Once it is detected that the raw material difluoromethane or the raw material dichloromethane are completely consumed, the feeding of the fluorine-containing olefin is stopped immediately. At this time, the total amount of the fluorine-containing olefin consequentially satisfies the above relationship. Thus, the polymerization of excessive amount of the fluorine-containing olefin at the active sites of the catalyst can be avoided, and the activity of the catalyst and the service life of the catalyst are not affected.

The content of fluorine-containing olefin in the reaction system is mainly monitored by subjecting a material consumption in the reaction system to chromatographic analysis.

In the method for preparing the fluorochloropropane of the present invention, after the metal fluoride, the metal chloride and the metal oxide are added in to a reactor (a reaction kettle or a tubular reactor) in a certain ratio, the dichloromethane is added into the reactor. The difluoromethane can be added into the reactor along with the dichloromethane; optionally, the difluoromethane can be continuously added into the reactor; optionally, the difluoromethane can be mixed with the fluorine-containing olefin to generate a mixed gas and continuously added in to the reactor.

Optionally, the addition catalyst and the dichloromethane are mixed together to obtain a uniform mixture and continuously added into the reactor; and the difluoromethane and the fluorine-containing olefin are mixed together and continuously added into the reactor or continuously added into the reactor, respectively.

In the method for preparing the fluorochloropropane of the present invention, any of the feeding methods described above can be applied, as long as the fluorine-containing olefin is continuously added into the reaction system. In some embodiments, the addition catalyst is prefilled in the reactor, then the dichloromethane and the difluoromethane can be added, and the fluorine-containing olefin is fed under pressure via a metering pump.

In the method for preparing the fluorochloropropane of the present invention, a temperature of the addition reaction is in a range of -20 degrees centigrade to 100 degrees centigrade, and a duration of the addition reaction is in a range of 4 hours to 20 hours. In some embodiments, the temperature of the addition reaction is in a range of 0 degrees centigrade to 80 degrees centigrade, and the duration of the addition reaction is in a range of 6 hours to 15 hours. In some embodiments, the temperature of the addition reaction is in a range of 30 degrees centigrade to 50 degrees centigrade, and the duration of the addition reaction is in a range of 8 hours to 12 hours.

The pressure invention, it is not necessary to control a reaction pressure, as long as the reactor is ensured to be sealed. The reaction pressure is determined by a saturated vapor pressure of the difluoromethane in the reactor, and is generally around 2 MPa. During the reaction, the reaction pressure gradually reduces while the difluoromethane consumes.

Since the saturated vapor pressure of the difluoromethane and saturated vapor pressure of the fluorine-containing olefin are much greater than that of the dichloromethane, the dichloromethane are mainly in the liquid state in the reaction process. In addition, in some embodiments, as the fluorochloropropane generated in the addition reaction is in liquid state, the addition reaction of the present invention is a solvent-free reaction, and it is not necessary to add other solvents into the reaction system during the reaction process. Thus, separation process of the products can be reduced.

The addition product obtained after the reaction is subjected to filtration and the addition catalyst and the fluorochloropropane can be effectively separated. In the obtained fluorochloropropane, a difference between a boiling point of a target product CF₃CR₁R₂CH₂Cl and a by-product CF₂ClCR₁R₂CH₂Cl is in a range of 15 degrees centigrade and 30 degrees centigrade. Thus, a fluorochloropropane intermediate having a purity greater than or equal to 99% can be obtained by distillation or rectification.

The present invention further provides a method for preparing a fluorine-containing propylene, which includes following steps:
step (1), preparing a fluorochloropropane intermediate by any of the methods described above;
step (2), subjecting the fluorochloropropane intermediate to a dehydrochlorination reaction under the action of activated carbon or a metal alkali solution to obtain the fluorine-containing propylene.

Step (1) further includes following steps: preparing a fluorochloropropane liquid by any of the methods described above, and subjecting the fluorochloropropane liquid to solid-liquid separation to remove the addition catalyst, and subjecting the fluorochloropropane liquid after the solid-liquid separation to rectification to obtain the fluorochloropropane intermediate.

In some embodiments, step (2) is a gas-phase dehalogenation reaction. In some embodiments, step (2) is a liquid-phase dehalogenation reaction.

In some embodiments, the gas-phase dehalogenation reaction is performed under the action of activated carbon, a temperature of the dehydrochlorination reaction is in a range of 200 degrees centigrade to 500 degrees centigrade. In some embodiments, the temperature of the dehydrochlorination reaction is in a range of 300 degrees centigrade to 350 degrees centigrade. In some embodiments, an activated carbon catalyst is selected from the group consisting of nut shell activated carbon, coal-based activated carbon, wood-based activated carbon, and any combination thereof. In some embodiments, the activated carbon catalyst is the nut shell activated carbon.

In some embodiments, the liquid-phase dehalogenation reaction is performed under the action of the metal alkali solution. In some embodiments, the liquid-phase dehalogenation reaction is performed under the action of the metal alkali solution and a quaternary ammonium salt. A molar ratio of the fluorochloropropane intermediate to a metal alkali is in a range of 1 : 0.1 to 1: 5. In some embodiments, the molar ratio of the fluorochloropropane intermediate to a metal alkali is in a range of 1 : 1 to 1: 2. A temperature of the dehydrochlorination reaction is in a range of 10 degrees centigrade to 100 degrees centigrade. In some embodiments, the temperature of the dehydrochlorination reaction is in a range of 30 degrees centigrade to 60 degrees centigrade. The metal alkali solution is selected from the group consisting of a sodium hydroxide solution, a potassium hydroxide solution, a sodium carbonate solution, a potassium carbonate solution, a sodium bicarbonate solution, a potassium bicarbonate solution, and any combination thereof, and a mass concentration of the metal alkali solution is in a range of 5 wt% to 60 wt%. In some embodiments, the metal alkali solution is selected from a sodium hydroxide solution or a potassium hydroxide solution. In some embodiments, the metal alkali solution is a potassium hydroxide solution. In some embodiments, the mass concentration of the metal alkali solution is in a range of 10 wt% to 40 wt%. The quaternary ammonium salt is selected from the group consisting of tetrabutylammonium bromide, tetramethylammonium hydroxide, tetramethylammonium chloride, and any combination thereof. In some embodiments, the quaternary ammonium salt is tetramethylammonium hydroxide.

In some embodiments, the method for preparing the fluorine-containing propylene includes following steps.

Steps of liquid-phase addition reaction: 1) subjecting dichloromethane, difluoromethane and fluorine-containing olefin to a liquid-phase addition reaction under the action of an addition catalyst, wherein the fluorine-containing olefin is selected from the group consisting of vinylidene fluoride, chlorodifluoroethylene, trifluoroethylene, chlorotrifluoroethylene, and any combination thereof; and 2) after the addition reaction, collecting unreacted gas-phase material, and subjecting a reaction liquid in the reaction kettle to solid-liquid separation, wherein a solid part obtained after the solid-liquid separation is the addition catalyst, and a liquid part obtained after the solid-liquid separation is subjected to rectification to obtain the fluorochloropropane intermediate; and the unreacted gas-phase material and the addition catalyst can be reused.

Steps of gas-phase/liquid-phase dehydrochlorination reaction: 3) filling an activated carbon catalyst in a second reactor, optionally, adding a metal alkali solution in the second reactor, optionally, adding a mixture of a metal alkali solution and a quaternary ammonium salt in the second reactor; 4) setting a corresponding reaction temperature, adding the fluorochloropropane intermediate into the second reactor and subjecting the fluorochloropropane intermediate to a gas-phase/liquid-phase dehydrochlorination reaction; and 5) subjecting a reactant to alkali washing process and neutralizing, subjecting the resultant to rectification to obtain a fluorine-containing propylene product.

Compared with the prior art, the present invention has the following beneficial effects.
1. The present invention requires only one-step addition reaction to prepare fluorochloropropane, with a simple technological process, mild reaction conditions, and high product yield.
2. The present invention, by monitoring the content of the fluorine-containing olefin in the reaction system and adjusting the feeding rate accordingly, the polymerization of the fluorine-containing olefin on active sites is inhibited, and the service life of the catalyst is significantly prolonged.
3. The three-component liquid-phase addition process of the present invention has simple post-treatment operations, easy product separation, and high product purity, making it highly suitable for industrial application.

### DETAILED DESCRIPTION

The present invention will be further illustrated in conjunction with the specific embodiments below, but the present invention is not limited to these particular embodiments. Those skilled in the art should recognize that the present invention encompasses all alternative, modified, and equivalent forms within the scope of the claims.

In a first aspect, the present invention provides embodiments for preparing a fluorochloropropane intermediate and comparative embodiments for preparing a fluorochloropropane intermediate.

### Embodiment 1

The present embodiment provided a method for preparing a 1-chloro-3,3,3-trifluoropropane, which included following steps.

20.2 g (0.24 mol) of anhydrous aluminum trifluoride, 18.7 g (0.14 mol) of anhydrous aluminum trichloride and 6.1 g (0.06 mol) of aluminum oxide were weighed and added into a reaction kettle. The reaction kettle was sealed and subjected to vacuum treatment. Then, 225 g of dichloromethane was added into the reaction kettle via a sampling device, and the reaction kettle was placed in a cooling circulation bath controlled by a cooling circulation pump at -30 degrees centigrade and reacted for 30 min. The reaction kettle was taken out of the cooling circulation pump, and 125 g of difluoromethane was continuously added into the reaction kettle. The vinylidene fluoride was added in the reaction kettle at a flow rate of 60 g/h under pressure via a metering pump, and a duration of the feeding process was 5 hours. After the feeding process, the raw materials reacted under conditions of a stirring rate of 300 rpm at 30 degrees centigrade for 5 hours.

During the reaction, variation of reaction pressure in the reaction kettle and variation of the temperature in the reaction kettle were recorded, and a content of the vinylidene fluoride in the reaction kettle was monitored at the same time. Specifically, sample was collected every 30 minutes, and the sample was tested by gas-phase chromatographic analysis. The results showed that in the whole process, the content of the vinylidene fluoride in the reaction system was smaller than or equal to 2.8%. After the reaction completed and the temperature in the reaction kettle lowered to room temperature, a gas-phase valve was opened to collect gas-phase materials (i.e., unreacted difluoromethane and a small amount of addition product). A liquid-phase material in the reaction kettle was filtered, and a solid part was the addition catalyst. A filter liquor was subjected to rectification to obtain a target product 1-chloro-3,3,3-trifluoropropane, and the purity of the target product 1-chloro-3,3,3-trifluoropropane was 99.7%.

The liquid-phase material and the gas-phase material obtained in the addition reaction were subjected to chromatographic analysis. It was calculated that a conversion rate of the vinylidene fluoride was 96.2%, a selectivity of 1-chloro-3,3,3-trifluoropropane was 85.6%, and a selectivity of a by-product 1,3-dichloro-1,1-difluoropropane was 13.7%. A small amount of other by-products was produced, and the by-product was identified by GC-MS analysis and as an isomer of 1-chloro-3,3,3-trifluoropropane.

### Embodiment 2

The present embodiment was substantially the same as embodiment 1, except the following steps. Firstly, the feeding rate of the vinylidene fluoride was improved to 100 g/h, and the duration of the feeding process of the vinylidene fluoride was 1 hour. Then, the feeding rate of difluoromethane was lowered to 50 g/h, and the duration of feeding process of difluoromethane was 4 hours. After the feeding processes, the reaction under conditions of stirring lasted for 5 hours.

Variation of the content of vinylidene fluoride in the reaction kettle was monitored, and the content of vinylidene fluoride increased as the reaction progressed. After reacted for 1 hour, the content of vinylidene fluoride in the reaction system reached 4.1%. Then, the feeding rate of vinylidene fluorine was reduced to 50 g/h, and the content of vinylidene fluorine in the reaction kettle gradually reduced to 0.8%.

The liquid-phase material and the gas-phase material obtained in the addition reaction were subjected to chromatographic analysis. It was calculated that a conversion rate of the vinylidene fluoride was 94.4%, a selectivity of 1-chloro-3,3,3-trifluoropropane was 83.5%, and a selectivity of a by-product 1,3-dichloro-1,1-difluoropropane was 15.8%. A small amount of other by-products was produced, and the by-product was identified by GC-MS analysis and as an isomer of 1-chloro-3,3,3-trifluoropropane. The product 1-chloro-3,3,3-trifluoropropane obtained after rectification had a purity of 99.6%.

### Embodiment 3

The present embodiment was substantially the same as embodiment 1, except the following steps. Firstly, the feeding rate of the vinylidene fluoride was improved to 90 g/h, and the duration of the feeding process of the vinylidene fluoride was 2 hours. Then, the feeding rate of vinylidene fluoride was lowered to 40 g/h, and the duration of feeding process of difluoromethane was 3 hours. After the feeding processes, the reaction under conditions of stirring lasted for 5 hours.

Variation of the content of vinylidene fluoride in the reaction kettle was monitored, and the content of vinylidene fluoride increased as the reaction progressed. After reacted for 2 hours, the content of vinylidene fluoride in the reaction system reached 4.8%. Then, the feeding rate of vinylidene fluorine was reduced to 40 g/h, and the content of vinylidene fluorine in the reaction kettle gradually reduced to 1.3%.

The liquid-phase material and the gas-phase material obtained in the addition reaction were subjected to chromatographic analysis. It was calculated that a conversion rate of the vinylidene fluoride was 88.4%, a selectivity of 1-chloro-3,3,3-trifluoropropane was 79.3%, and a selectivity of a by-product 1,3-dichloro-1,1-difluoropropane was 19.7%. A small amount of other by-products was produced, and the by-product was identified by GC-MS analysis and as an isomer of 1-chloro-3,3,3-trifluoropropane. The product 1-chloro-3,3,3-trifluoropropane obtained after rectification had a purity of 99.7%.

### Embodiment 4

The present embodiment was substantially the same as embodiment 1, except for that the addition catalyst was 48.4 g (0.19 mol) of anhydrous hafnium tetrafluoride, 41.9 g (0.14 mol) of anhydrous antimony pentachloride and 12.2 g (0.12 mol) of aluminum oxide. Other conditions were the same.

The liquid-phase material and the gas-phase material obtained in the addition reaction were subjected to chromatographic analysis. It was calculated that a conversion rate of the vinylidene fluoride was 86.4%, a selectivity of 3-chloro-1,1,1-trifluoropropane was 76.7%, and a selectivity of a by-product 1,3-dichloro-1,1-difluoropropane was 23.0%. A small amount of other by-products was produced, and the by-product was identified by GC-MS analysis and as an isomer of 3-chloro-1,1,1-trifluoropropane.

### Embodiment 5

The present embodiment was substantially the same as embodiment 1, except the following. The addition catalyst was 39.4 g (0.21 mol) of anhydrous niobium pentafluoride, 39.6 g (0.18 mol) of anhydrous zirconium tetrachloride, and 30.5 g (0.11 mol) of indium(III) oxide, and other conditions were the same.

The liquid-phase material and the gas-phase material obtained in the addition reaction were subjected to chromatographic analysis. It was calculated that a conversion rate of the vinylidene fluoride was 76.1%, a selectivity of 3-chloro-1,1,1-trifluoropropane was 69.8%, and a selectivity of a by-product 1,3-dichloro-1,1-difluoropropane was 29.3%. A small amount of other by-products was produced, and the by-product was identified by GC-MS analysis and as an isomer of 3-chloro-1,1,1-trifluoropropane.

### Embodiment 6

The present embodiment was substantially the same as embodiment 1, except the following. The addition catalyst was 47.6 g (0.22 mol) of anhydrous antimony pentafluoride, 24.7 g (0.13 mol) of anhydrous titanium tetrachloride, and 26.5 g (0.06 mol) of tantalum pentoxide. Other conditions were the same.

The liquid-phase material and the gas-phase material obtained in the addition reaction were subjected to chromatographic analysis. It was calculated that a conversion rate of the vinylidene fluoride was 83.7%, a selectivity of 3-chloro-1,1,1-trifluoropropane was 76.2%, and a selectivity of a by-product 1,3-dichloro-1,1-difluoropropane was 23.2%. A small amount of other by-products was produced, and the by-product was identified by GC-MS analysis and as an isomer of 3-chloro-1,1,1-trifluoropropane.

### Embodiments 7 to 12

Embodiments 7 to 12 were substantially the same as Embodiment 1, except the following. An amount of anhydrous aluminum trifluoride, an amount of anhydrous aluminum trichloride and an amount of aluminum oxide in the mixed catalyst were adjusted to control the content of the fluorine element, the content of the chloride element and the content of the oxygen element. The specific results were shown in Table 1.

**Table 1 Results of addition reaction under the action of catalysts containing varying amounts of fluorine, chlorine, and oxygen.**

| Embodiment | Content of fluorine element % | Content of chlorine element % | Content of oxygen element % | Conversion rate of vinylidene fluoride /% | Selectivity of 1-chloro-3,3,3 - trifluoropropane |
|---|---|---|---|---|---|
| 7 | 40.0 | 30.0 | 30.0 | 84.5 | 87.4 |
| 8 | 45.0 | 30.0 | 25.0 | 92.1 | 86.2 |
| 9 | 40.0 | 50.0 | 10.0 | 86.6 | 73.8 |
| 10 | 60.0 | 25.0 | 15.0 | 90.2 | 82.6 |
| 11 | 47.5 | 40.0 | 12.5 | 88.7 | 85.4 |
| 12 | 70.0 | 20.0 | 10.0 | 93.3 | 75.7 |

### Embodiments 13 to 28

Embodiment 13 to 28 were substantially the same as Embodiment 1, except that the type of metal cation of the addition catalyst was changed, the composition of fluorine element, chlorine element and oxygen element remained unchanged, and the reaction temperature was adjusted to match the catalyst activity. The specific results were shown in Table 2.

**Table 2 Results of addition reaction under addition catalysts including different metal cations**

| Embodiment | Reaction temperature /°C | Metal cations | Conversion rate of vinylidene fluoride /% | Selectivity of 1-chloro-3,3,3-trifluoropropane /% |
|---|---|---|---|---|
| 13 | 30 | Ti⁴⁺ | 82.3 | 76.5 |
| 14 | 65 | Ti⁴⁺ | 90.6 | 84.8 |
| 15 | 30 | Sb⁵⁺ | 86.3 | 81.3 |
| 16 | 55 | Sb⁵⁺ | 92.7 | 85.8 |
| 17 | 30 | Zr⁴⁺ | 86.2 | 79.3 |
| 18 | 40 | Zr⁴⁺ | 93.8 | 86.6 |
| 19 | 30 | Hf⁴⁺ | 76.3 | 73.5 |
| 20 | 50 | Hf⁴⁺ | 94.6 | 88.4 |
| 21 | 30 | Nb⁵⁺ | 86.4 | 78.2 |
| 22 | 40 | Nb⁵⁺ | 91.1 | 82.5 |
| 23 | 30 | Ta⁵⁺ | 73.9 | 80.4 |
| 24 | 40 | Ta⁵⁺ | 84.7 | 80.8 |
| 25 | 30 | Ga³⁺ | 80.6 | 82.1 |
| 26 | 50 | Ga³⁺ | 92.6 | 77.3 |
| 27 | 30 | In³⁺ | 87.2 | 82.5 |
| 28 | 45 | In³⁺ | 92.5 | 85.2 |

### Embodiment 29 to 38

Embodiments 29 to 38 were substantially the same as Embodiment 1, except that the conditions of the reaction were changed, the added amount of addition catalyst was not changed, the feeding rate of vinylidene fluoride was not changed, and the molar ratio of difluoromethane to vinylidene fluoride was not changed. The specific results were shown in Table 3.

**Table 3 Results of the addition reaction under different conditions**

| Embodiment | Reaction temperature /°C | Total reaction duration /h | Weight ratio of dichloro-methane to addition catalyst | Molar ratio of dichloro-methane to vinylidene fluoride | Vinylidene fluoride conversion rate /% | Selectivity of 1-chloro-3,3,3-trifluoropropane |
|---|---|---|---|---|---|---|
| 29 | 20 | 6 | 5 | 2:1 | 89.6 | 83.5 |
| 30 | -20 | 15 | 5 | 0.5:1 | 66.8 | 74.2 |
| 31 | 30 | 4 | 8 | 0.5:1 | 95.2 | 88.7 |
| 32 | 40 | 12 | 4 | 1:1 | 98.8 | 81.8 |
| 33 | 0 | 20 | 2 | 0.6:1 | 81.3 | 80.6 |
| 34 | 100 | 10 | 2 | 0.8:1 | 89.7 | 78.4 |
| 35 | 80 | 12 | 10 | 0.8:1 | 92.2 | 83.7 |
| 36 | 50 | 10 | 8 | 0.6:1 | 91.8 | 85.2 |
| 37 | 60 | 4 | 4 | 0.7:1 | 78.4 | 80.9 |
| 38 | 20 | 8 | 2 | 0.5:1 | 97.1 | 84.2 |

### Embodiments 39 to 50

Embodiments 39 to 50 were substantially the same as Embodiment 1, except that the type of the fluorine-containing olefin was changed to prepare different types of fluorochloropropane, and the reaction temperature and the total reaction duration were changed. The specific results were shown in Table 4.

**Table 4 Results of addition reaction with different raw materials**

| Embodiment | Reaction temperature /°C | Total reaction duration /h | Fluorine-containing olefin | Product fluoro chloropropane | Conversion rate of fluorine-containing olefin /% | Selectivity of addition product /% |
|---|---|---|---|---|---|---|
| 39 | 30 | 10 | CF₂=CHCl | CF₃CHClCH₂Cl | 94.6 | 87.5 |
| 40 | 10 | 15 | CF₂=CHCl | CF₃CHClCH₂Cl | 86.4 | 82.6 |
| 41 | 50 | 8 | CF₂=CHCl | CF₃CHClCH₂Cl | 97.2 | 89.2 |
| 42 | 30 | 10 | CF₂=CHF | CF₃CHFCH₂Cl | 98.3 | 90.7 |
| 43 | 40 | 8 | CF₂=CHF | CF₃CHFCH₂Cl | 98.7 | 87.4 |
| 44 | 50 | 15 | CF₂=CHF | CF₃CHFCH₂Cl | 97.4 | 89.3 |
| 45 | 50 | 6 | CF₂=CHF | CF₃CHFCH₂Cl | 89.7 | 83.8 |
| 46 | 80 | 8 | CF₂=CHF | CF₃CHFCH₂Cl | 85.5 | 79.2 |
| 47 | 40 | 12 | CF₂=CHF | CF₃CHFCH₂Cl | 99.5 | 91.6 |
| 48 | 30 | 10 | CF₂=CFCl | CF₃CFClCH₂Cl | 98.9 | 85.8 |
| 49 | 50 | 12 | CF₂=CFCl | CF₃CFClCH₂Cl | 96.7 | 83.5 |
| 50 | 20 | 10 | CF₂=CFCl | CF₃CFClCH₂Cl | 87.1 | 76.2 |

### Embodiment 51 to 53

Embodiment 51 to 53 were catalyst reuse experiments of the addition catalyst in the preparation process of 1-chloro-3,3,3-trifluoropropane in Examples 1 to 3, respectively. The specific results were shown in Table 5.

**Table 5 Results of catalyst reuse experiments with different amounts of vinylidene fluoride**

| Embodiment | Number of reuses/ time | Conversion rate of vinylidene fluoride /% | Selectivity of 1-chloro-3,3,3-trifluoropropane /% | Total catalytic efficiency / g_{product} *g_{cat.}⁻¹ |
|---|---|---|---|---|
| | 1 | 95.7 | 85.8 | 23.1 |
| | 2 | 94.1 | 84.5 | 34.0 |
| 51 | 3 | 94.3 | 85.2 | 45.1 |
| | 4 | 93.2 | 84.7 | 56.0 |
| | 5 | 90.6 | 83.6 | 66.5 |
| | 6 | 85.8 | 82.2 | 76.2 |
| 52 | 1 | 91.6 | 81.3 | 21.5 |
| | 2 | 87.2 | 80.6 | 31.2 |
| | 3 | 81.4 | 76.3 | 39.8 |
| | 4 | 70.5 | 72.5 | 46.8 |
| | 5 | 54.2 | 69.4 | 52.0 |
| 53 | 1 | 84.5 | 79.3 | 19.2 |
| | 2 | 79.1 | 76.3 | 27.6 |
| | 3 | 70.6 | 71.5 | 34.5 |
| | 4 | 59.6 | 63.5 | 39.8 |
| | 5 | 37.2 | 52.6 | 42.5 |

Comment: in the table, the number of reuses referred to the number of times the catalyst has been repeatedly evaluated. The number of reuses for the first use of the catalyst was 0, and for the second use was 1.

### Comparative embodiments 1 to 6

Methods of comparative embodiments 1 to 6 were the same as those in Embodiment 1, except that the addition catalyst was changed to anhydrous aluminum trifluoride, or anhydrous aluminum trichloride, or aluminum oxide, or a combination of any two of anhydrous aluminum trifluoride, anhydrous aluminum trichloride and aluminum oxide; the content of fluorine element, the content of chloride element and the content of oxygen element were adjusted by changing the amount of the addition catalyst; and other reaction conditions were not changed. The specific results were shown in Table 6.

**Table 6 Results of addition reaction using catalysts with different element contents**

| Comparative embodiment | Content of fluorine element % | Content of chlorine element % | Content of oxygen element % | Conversion rate of vinylidene fluoride /% | Selectivity of 1-chloro-3,3,3 - trifluoropropane /% |
|---|---|---|---|---|---|
| 1 | 100 | 0 | 0 | 23.6 | 31.7 |
| 2 | 0 | 100 | 0 | 15.3 | 19.7 |
| 3 | 0 | 0 | 100 | 2.8 | 12.9 |
| 4 | 50.0 | 50.0 | 0 | 72.5 | 47.3 |
| 5 | 60 | 0 | 40 | 46.1 | 57.2 |
| 6 | 0 | 70.0 | 30.0 | 16.6 | 21.3 |

### Comparative embodiment 7

Method of comparative embodiment 7 was substantially the same as that in Embodiment 1, except that composition of the fluorine element, the chloride element and the oxygen element in the addition catalyst were not changed, a total amount of the addition catalyst was reduced to 1 g, an amount of raw material dichloromethane was increased to 600 g, an amount of raw material vinylidene fluoride was increased to 750 g, an amount of raw material difluoromethane was added to 200 g. Other conditions were not changed.

The liquid-phase material and the gas-phase material obtained in the addition reaction were subjected to chromatographic analysis. It was calculated that a conversion rate of the vinylidene fluoride was only 8.9%, a selectivity of 1-chloro-3,3,3-trifluoropropane was 43.5%, and a selectivity of a by-product 1,3-dichloro-1,1-difluoropropane was 55.8%.

### Comparative embodiment 8

Method of comparative embodiment 8 was substantially the same as that in Embodiment 1, except that a total amount of raw material dichloromethane was increased to 800 g, an amount of raw material vinylidene fluoride was reduced to 20 g, an amount of raw material difluoromethane was added to 200 g, and other conditions were not changed.

The liquid-phase material and the gas-phase material obtained in the addition reaction were subjected to chromatographic analysis. It was calculated that a conversion rate of the vinylidene fluoride was 99.7%, a selectivity of 1-chloro-3,3,3-trifluoropropane was 39.5%, and a selectivity of a by-product 1,3-dichloro-1,1-difluoropropane was 59.8%.

### Comparative embodiment 9

Method of comparative embodiment 9 was substantially the same as that in Embodiment 1, except that a method for feeding the raw materials were changed. 300 g of vinylidene fluoride was prefilled in the reaction kettle, the dichloromethane and the difluoromethane were fed via a metering pump, a feeding rate of the dichloromethane was 45 g/h, a feeding rate of the difluoromethane was 25 g/h, a total amount of the dichloromethane was 225 g, and a total of the difluoromethane was 125 g.

The liquid-phase material and the gas-phase material obtained in the addition reaction were subjected to chromatographic analysis. It was calculated that a conversion rate of the vinylidene fluoride was 48.5%, a selectivity of 1-chloro-3,3,3-trifluoropropane was 52.7%, and a selectivity of a by-product 1,3-dichloro-1,1-difluoropropane was 46.4%.

### Comparative embodiment 10

Method of comparative embodiment 10 was substantially the same as that in Embodiment 1, except that the feeding rate of vinylidene fluoride was constant at 150 g/h, the duration of feeding process was 2 hours. After the feeding process, the reaction system was stirred and reacted for 7 h, and a catalyst reuse experiment was carried out under such reaction conditions. The specific results were shown in Table 6.

**Table 7 Results of catalyst reuse experiments with different feeding rates**

| Comparative embodiment | Number of reuses/ time | Content of vinylidene fluoride after the feeding process /% | Vinylidene fluoride conversion rate /% | Selectivity of 1-chloro-3,3,3-trifluoropropane /% | Total catalytic efficiency / g_{product} *g_{cat.}⁻¹ |
|---|---|---|---|---|---|
| | 0 | 8.6 | 79.2 | 73.5 | 8.3 |
| 10 | 1 | 13.5 | 64.2 | 69.3 | 14.4 |
| | 2 | 21.8 | 45.2 | 52.8 | 17.7 |
| | 3 | 29.3 | 19.2 | 26.2 | 18.4 |

In a second aspect of the present invention, a method for preparing a fluorine-containing propylene is provided.

### Embodiment 54

The present embodiment provided a method for preparing a 3,3,3-trifluoropropene. The 3-chloro-1,1,1-trifluoropropane (having a purity of 99.7%) obtained in Embodiment 1 was used as the raw material and subjected to a gas-phase dehydrochlorination reaction. The specific steps were shown hereinafter.

An Inconel alloy tubular reactor was used as a second reactor, an inner size of the tubular reactor was 19 mm, and a length of the tubular reactor was 800 mm. 20 mL of coconut shell activated carbon having a size in a range of 10 meshes to 20 meshes was filled to the middle of the second reactor. The tubular reactor was charged, and swept with nitrogen at a flow rate of 100 mL/min. A reaction temperature was set as 350 degrees centigrade, a heating rate was 5 degrees centigrade/min, and the reaction furnace started heating. When a temperature of a catalyst bed reached the reaction temperature, the flowing rate of nitrogen was adjusted to 20 mL/min, and 3-chloro-1,1,1-trifluoropropane (having a purity of 99.7%) was continuously added into the fixed-bed reactor at a feeding rate of 5.0 g/h to react.

A mixed gas flowing out of the reactor was analyzed by online GC analysis and GC/MS analysis, a conversion rate of the 3-chloro-1,1,1-trifluoropropane was 99.6%, and a selectivity of the product 3,3,3-trifluoropropene was 99.3%.

### Embodiment 55

The present embodiment provided a method for preparing a 2-chloro-3,3,3-trifluoropropene, which included an addition process and a dehydrochlorination process. The specific steps were shown hereinafter.

### (1) Additional process

20.2 g (0.24 mol) of anhydrous aluminum trifluoride, 18.7 g (0.14 mol) of anhydrous aluminum trichloride and 6.1 g (0.06 mol) of aluminum oxide were weighed and added to the reaction kettle. The reaction kettle was sealed and subjected to vacuum treatment. Then, 225 g of dichloromethane was added into the reaction kettle via a sampling device, and the reaction kettle was placed in a cooling circulation bath controlled by a cooling circulation pump at -30 degrees centigrade and reacted for 30 min. The reaction kettle was taken out of the cooling circulation pump, and 125 g of difluoromethane was continuously added into the reaction kettle. The chlorodifluoroethylene was added in the reaction kettle at a flow rate of 120 g/h under pressure via a metering pump, and a duration of the feeding process was 5 hours. After the feeding process, the raw materials reacted under conditions of a stirring rate of 300 rpm at 30 degrees centigrade for 5 hours.

During the reaction, variation of reaction pressure in the reaction kettle and variation of the temperature in the reaction kettle were recorded, and a content of the chlorodifluoroethylene in the reaction kettle was monitored at the same time. Specifically, sample was collected every 30 minutes, and the sample was tested by gas-phase chromatographic analysis. The results showed that in the whole process, the content of the chlorodifluoroethylene in the reaction system was smaller than or equal to 2.6%. After the reaction completed and the temperature in the reaction kettle lowered to room temperature, a gas-phase valve was opened to collect gas-phase materials (i.e., unreacted difluoromethane and a small amount of addition product). A liquid-phase material in the reaction kettle was filtered, and a solid part was the addition catalyst. A filter liquor was subjected to rectification to obtain the 2,3-dichloro-1,1,1-trifluoropropane, and the purity of the 2,3-dichloro-1,1,1-trifluoropropane was 99.6%.

The liquid-phase material and the gas-phase material obtained in the addition reaction were subjected to chromatographic analysis. It was calculated that a conversion rate of the chlorodifluoroethylene was 95.3%, a selectivity of 2,3-dichloro-1,1,1-trifluoropropane was 84.5%, and a selectivity of a by-product 1,2,3-trichloro-1,1-difluoropropane was 14.7%. A small amount of other by-products was produced, and the by-product was identified by GC-MS analysis and as an isomer of 2,3-dichloro-1,1,1-trifluoropropane.

### (2) Dehydrochlorination process

An Inconel alloy tubular reactor was used as a second reactor, an inner size of the tubular reactor was 19 mm, and a length of the tubular reactor was 800 mm. 20 mL of coconut shell activated carbon having a size in a range of 10 meshes to 20 meshes was filled to the middle of the second reactor. The tubular reactor was charged, and swept with nitrogen at a flow rate of 100 mL/min. A reaction temperature was set as 350 degrees centigrade, a heating rate was 5 degrees centigrade/min, and the reaction furnace started heating. When a temperature of a catalyst bed reached the reaction temperature, the flowing rate of nitrogen was adjusted to 20 mL/min, and 2,3-dichloro-1,1,1-trifluoropropane (having a purity of 99.6%) was continuously added into the second reactor at a feeding rate of 5.0 g/h to react.

A mixed gas flowing out of the reactor was analyzed by online GC analysis and GC/MS analysis, a conversion rate of the 2,3-dichloro-1,1,1-trifluoropropane was 99.4%, and a selectivity of the product 2-chloro-3,3,3-trifluoropropene was 97.1%.

### Embodiment 56

The present embodiment provided a method for preparing a 2,3,3,3-tetrafluoropropene, which included an addition process and a dehydrochlorination process. The specific steps were shown hereinafter.

### (1) Additional process

20.2 g (0.24 mol) of anhydrous aluminum trifluoride, 18.7 g (0.14 mol) of anhydrous aluminum trichloride and 6.1 g (0.06 mol) of aluminum oxide were weighed and added to the reaction kettle. The reaction kettle was sealed and subjected to vacuum treatment. Then, 225 g of dichloromethane was added into the reaction kettle via a sampling device, and the reaction kettle was placed in a cooling circulation bath controlled by a cooling circulation pump at -30 degrees centigrade and reacted for 30 min. The reaction kettle was taken out of the cooling circulation pump, and 125 g of difluoromethane was continuously added into the reaction kettle. The trifluoroethylene was added in the reaction kettle at a flow rate of 75 g/h under pressure via a metering pump, and a duration of the feeding process was 5 hours. After the feeding process, the raw materials reacted under conditions of a stirring rate of 300 rpm at 30 degrees centigrade for 5 hours.

During the reaction, variation of reaction pressure in the reaction kettle and variation of the temperature in the reaction kettle were recorded, and a content of the trifluoroethylene in the reaction kettle was monitored at the same time. Specifically, sample was collected every 30 minutes, and the sample was tested by gas-phase chromatographic analysis. The results showed that in the whole process, the content of the trifluoroethylene in the reaction system was smaller than or equal to 2.5%. After the reaction completed and the temperature in the reaction kettle lowered to room temperature, a gas-phase valve was opened to collect gas-phase materials (i.e., unreacted difluoromethane and a small amount of addition product). A liquid-phase material in the reaction kettle was filtered, and a solid part was the addition catalyst. A filter liquor was subjected to rectification to obtain the 3-chloro-1,1,1,2-tetrafluoropropane, and the purity of the 3-chloro-1,1,1,2-tetrafluoropropane was 99.8%.

The liquid-phase material and the gas-phase material obtained in the addition reaction were subjected to chromatographic analysis. It was calculated that a conversion rate of the trifluoroethylene was 98.6%, a selectivity of 3-chloro-1,1,1,2-tetrafluoropropane was 88.5%, and a selectivity of a by-product 1,3-dichloro-1,1,2-trifluoropropane was 9.4%. A small amount of other by-products was produced, and the by-product was identified by GC-MS analysis and as an isomer of 3-chloro-1,1,1,2-tetrafluoropropane.

### (2) Dehydrochlorination process

An Inconel alloy tubular reactor was used as a second reactor, an inner size of the tubular reactor was 19 mm, and a length of the tubular reactor was 800 mm. 20 mL of coconut shell activated carbon having a size in a range of 10 meshes to 20 meshes was filled to the middle of the second reactor. The tubular reactor was charged, and swept with nitrogen at a flow rate of 100 mL/min. A reaction temperature was set as 350 degrees centigrade, a heating rate was 5 degrees centigrade/min, and the reaction furnace started heating. When a temperature of a catalyst bed reached the reaction temperature, the flowing rate of nitrogen was adjusted to 20 mL/min, and 3-chloro-1,1,1,2-tetrafluoropropane (having a purity of 99.8%) was continuously added into the second reactor at a feeding rate of 5.0 g/h to react.

A mixed gas flowing out of the reactor was analyzed by online GC analysis and GC/MS analysis, a conversion rate of the 3-chloro-1,1,1,2-tetrafluoropropane was 99.0%, and a selectivity of the product 2,3,3,3-tetrafluoropropene was 99.2%.

### Embodiment 57

The present embodiment provided a method for preparing a 1-chloro-2,3,3,3-tetrafluoropropene, which included an addition process and a dehydrochlorination process. The specific steps were shown hereinafter.

### (1) Additional process

20.2 g (0.24 mol) of anhydrous aluminum trifluoride, 18.7 g (0.14 mol) of anhydrous aluminum trichloride and 6.1 g (0.06 mol) of aluminum oxide were weighed and added to the reaction kettle. The reaction kettle was sealed and subjected to vacuum treatment. Then, 225 g of dichloromethane was added into the reaction kettle via a sampling device, and the reaction kettle was placed in a cooling circulation bath controlled by a cooling circulation pump at -30 degrees centigrade and reacted for 30 min. The reaction kettle was taken out of the cooling circulation pump, and 125 g of difluoromethane was continuously added into the reaction kettle. The chlorotrifluoroethylene was added in the reaction kettle at a flow rate of 100 g/h under pressure via a metering pump, and a duration of the feeding process was 5 hours. After the feeding process, the raw materials reacted under conditions of a stirring rate of 300 rpm at 30 degrees centigrade for 5 hours.

During the reaction, variation of reaction pressure in the reaction kettle and variation of the temperature in the reaction kettle were recorded, and a content of the chlorotrifluoroethylene in the reaction kettle was monitored at the same time. Specifically, sample was collected every 30 minutes, and the sample was tested by gas-phase chromatographic analysis. The results showed that in the whole process, the content of the chlorotrifluoroethylene in the reaction system was smaller than or equal to 3.0%. After the reaction completed and the temperature in the reaction kettle lowered to room temperature, a gas-phase valve was opened to collect gas-phase materials (i.e., unreacted difluoromethane and a small amount of addition product). A liquid-phase material in the reaction kettle was filtered, and a solid part was the addition catalyst. A filter liquor was subjected to rectification to obtain the 2,3-dichloro-1,1,1,2-tetrafluoropropane, and the purity of the 2,3-dichloro-1,1,1,2-tetrafluoropropane was 99.5%.

The liquid-phase material and the gas-phase material obtained in the addition reaction were subjected to chromatographic analysis. It was calculated that a conversion rate of the chlorotrifluoroethylene was 97.1%, a selectivity of 2,3-dichloro-1,1,1,2-tetrafluoropropane was 79.8%, and a selectivity of a by-product 1,2,3-trichloro-1,1,2-trifluoropropane was 12.6%. A small amount of other by-products was produced, and the by-product was identified by GC-MS analysis and as an isomer of 2,3-dichloro-1,1,1,2-tetrafluoropropane.

### (2) Dehydrochlorination process

An Inconel alloy tubular reactor was used as a second reactor, an inner size of the tubular reactor was 19 mm, and a length of the tubular reactor was 800 mm. 20 mL of coconut shell activated carbon having a size in a range of 10 meshes to 20 meshes was filled to the middle of the second reactor. The tubular reactor was charged, and swept with nitrogen at a flow rate of 100 mL/min. A reaction temperature was set as 350 degrees centigrade, a heating rate was 5 degrees centigrade/min, and the reaction furnace started heating. When a temperature of a catalyst bed reached the reaction temperature, the flowing rate of nitrogen was adjusted to 20 mL/min, and 2,3-dichloro-1,1,1,2-tetrafluoropropane (having a purity of 99.5%) was continuously added into the second reactor at a feeding rate of 5.0 g/h to react.

A mixed gas flowing out of the reactor was analyzed by online GC analysis and GC/MS analysis, a conversion rate of the 2,3-dichloro-1,1,1,2-tetrafluoropropane was 95.9%, and a selectivity of the product 1-chloro-2,3,3,3-tetrafluoropropene was 98.7%.

### Embodiment 58

Method of the present embodiment was substantially the same as that in Embodiment 54, except that the coconut shell activated carbon was replaced by coal-based activated carbon having a size in a range of 10 meshes to 20 meshes. The other operations were not changed.

A product of the dehydrochlorination process was analyzed by online GC analysis and GC/MS analysis, a conversion rate of the 3-chloro-1,1,1-trifluoropropane was 99.2%, and a selectivity of the product 3,3,3-trifluoropropene was 95.1%.

### Embodiment 59

Method of the present embodiment was substantially the same as that in Embodiment 54, except that in the dehydrochlorination process, the reaction temperature was lowered to 300 degrees centigrade. The other operations were not changed.

A product of the dehydrochlorination process was analyzed by online GC analysis and GC/MS analysis, a conversion rate of the 3-chloro-1,1,1-trifluoropropane was 75.8%, and a selectivity of the product 3,3,3-trifluoropropene was 99.2%.

### Embodiment 60

Method of the present embodiment was substantially the same as that in Embodiment 54, except that in the dehydrochlorination process, the reaction temperature was lowered to 320 degrees centigrade. The other operations were not changed.

A product of the dehydrochlorination process was analyzed by online GC analysis and GC/MS analysis, a conversion rate of the 3-chloro-1,1,1-trifluoropropane was 86.9%, and a selectivity of the product 3,3,3-trifluoropropene was 99.1%.

### Embodiment 61

Method of the present embodiment was substantially the same as that in Embodiment 54, except that the dehydrochlorination process was performed by a liquid-phase process. The specific steps were shown hereinafter.

A 250 mL high-pressure reaction kettle made of Inconel alloy was used as a second reactor. 20 g of 3-chloro-1,1,1-trifluoropropane (having a purity of 99.7%), 40 g of KOH solution (25wt%), and 1 g of tetramethylammonium hydroxide were added in the reaction kettle. The reaction kettle was sealed, and 1.0 MPa nitrogen was introduced into the reaction kettle to replace the air in the reaction kettle. The replacement process repeated for three times. After the replacement process, the reaction temperature was set as 50 degrees centigrade, and the reaction performed under conditions of stirring at 300 rpm for 5 hours.

After the reaction, the reaction product was subjected to liquid separation and rectification to obtain the target product 3,3,3-trifluoropropene. The reaction product was analyzed by gas-phase chromatographic analysis. A conversion rate of 3-chloro-1,1,1-trifluoropropane was 90.8%, and a selectivity of the main product 3,3,3-trifluoropropene was 91.2%.

### Embodiment 62

Method of the present embodiment was substantially the same as that in Embodiment 61, except that in the dehydrochlorination process, the KOH solution (25 wt%) was replaced with 40 g of NaOH solution (25 wt%). The other operations were not changed.

A product of the dehydrochlorination process was analyzed by online GC analysis and GC/MS analysis, a conversion rate of the 3-chloro-1,1,1-trifluoropropane was 70.6%, and a selectivity of the main product 3,3,3-trifluoropropene was 81.6%.

### Embodiment 63

Method of the present embodiment was substantially the same as that in Embodiment 61, except that in the dehydrochlorination process, the reaction temperature increased to 80 degrees centigrade. The other operations were not changed.

A product of the dehydrochlorination process was analyzed by online GC analysis and GC/MS analysis, a conversion rate of the 3-chloro-1,1,1-trifluoropropane was 95.1%, and a selectivity of the main product 3,3,3-trifluoropropene was 80.8%.

### Embodiment 64

Method of the present embodiment was substantially the same as that in Embodiment 55, except that the coconut shell activated carbon was replaced by coal-based activated carbon having a size in a range of 10 meshes to 20 meshes. The other operations were not changed.

A product of the dehydrochlorination process was analyzed by online GC analysis and GC/MS analysis, a conversion rate of the 2,3-dichloro-1,1,1-trifluoropropane was 98.6%, and a selectivity of the product 2-chloro-3,3,3-trifluoropropene was 96.7%.

### Embodiment 65

Method of the present embodiment was substantially the same as that in Embodiment 55, except that in the dehydrochlorination process, the reaction temperature was lowered to 300 degrees centigrade. The other operations were not changed.

A product of the dehydrochlorination process was analyzed by online GC analysis and GC/MS analysis, a conversion rate of the 2,3-dichloro-1,1,1-trifluoropropane was 78.8%, and a selectivity of the product 2-chloro-3,3,3-trifluoropropene was 97.8%.

### Embodiment 66

Method of the present embodiment was substantially the same as that in Embodiment 55, except that in the dehydrochlorination process, the reaction temperature was lowered to 320 degrees centigrade. The other operations were not changed.

A product of the dehydrochlorination process was analyzed by online GC analysis and GC/MS analysis, a conversion rate of the 2,3-dichloro-1,1,1-trifluoropropane was 86.8%, and a selectivity of the product 2-chloro-3,3,3-trifluoropropene was 98.1%.

### Embodiment 67

Method of the present embodiment was substantially the same as that in Embodiment 55, except that the dehydrochlorination process was performed by a liquid-phase process. The specific steps were shown hereinafter.

A 250 mL high-pressure reaction kettle made of Inconel alloy was used as a second reactor. 20 g of 2,3-dichloro-1,1,1-trifluoropropane (having a purity of 99.6%), 40 g of KOH solution (25wt%), and 1 g of tetramethylammonium hydroxide were added in the reaction kettle. The reaction kettle was sealed, and 1.0 MPa nitrogen was introduced into the reaction kettle to replace the air in the reaction kettle. The replacement process repeated for three times. After the replacement process, the reaction temperature was set as 50 degrees centigrade, and the reaction performed under conditions of stirring at 300 rpm for 5 hours.

After the reaction, the reaction product was subjected to liquid separation and rectification to obtain the target product 2-chloro-3,3,3-trifluoropropene. The reaction product was analyzed by gas-phase chromatographic analysis. A conversion rate of 2,3-dichloro-1,1,1-trifluoropropane was 83.9%, and a selectivity of the product 2-chloro-3,3,3-trifluoropropene was 96.9%.

### Embodiment 68

Method of the present embodiment was substantially the same as that in Embodiment 67, except that in the dehydrochlorination process, the KOH solution (25 wt%) was replaced with 40 g of NaOH solution (25 wt%). The other operations were not changed.

A product of the dehydrochlorination process was analyzed by online GC analysis and GC/MS analysis, a conversion rate of the 2,3-dichloro-1,1,1-trifluoropropane was 70.8%, and a selectivity of the product 2-chloro-3,3,3-trifluoropropene was 93.6%.

### Embodiment 69

Method of the present embodiment was substantially the same as that in Embodiment 67, except that in the dehydrochlorination process, the reaction temperature increased to 80 degrees centigrade. The other operations were not changed.

A product of the dehydrochlorination process was analyzed by online GC analysis and GC/MS analysis, a conversion rate of the 2,3-dichloro-1,1,1-trifluoropropane was 89.7%, and a selectivity of the product 2-chloro-3,3,3-trifluoropropene was 92.9%.

### Embodiment 70

Operations of the present embodiment were the same as those in Embodiment 56, except that the coconut shell activated carbon was replaced by coal-based activated carbon having a size in a range of 10 meshes to 20 meshes. The other operations were not changed.

A product of the dehydrochlorination process was analyzed by online GC analysis and GC/MS analysis, a conversion rate of the 3-chloro-1,1,1,2-tetrafluoropropane was 95.0%, and a selectivity of the product 2,3,3,3-tetrafluoropropene was 99.1%.

### Embodiment 71

Method of the present embodiment was substantially the same as that in Embodiment 56, except that in the dehydrochlorination process, the reaction temperature was lowered to 300 degrees centigrade. The other operations were not changed.

A product of the dehydrochlorination process was analyzed by online GC analysis and GC/MS analysis, a conversion rate of the 3-chloro-1,1,1,2-tetrafluoropropane was 80.8%, and a selectivity of the product 2,3,3,3-tetrafluoropropene was 99.2%.

### Embodiment 72

Method of the present embodiment was substantially the same as that in Embodiment 56, except that in the dehydrochlorination process, the reaction temperature was lowered to 320 degrees centigrade. The other operations were not changed.

A product of the dehydrochlorination process was analyzed by online GC analysis and GC/MS analysis, a conversion rate of the 3-chloro-1,1,1,2-tetrafluoropropane was 85.9%, and a selectivity of the product 2,3,3,3-tetrafluoropropene was 99.1%.

### Embodiment 73

Method of the present embodiment was substantially the same as that in Embodiment 56, except that the dehydrochlorination process was performed by a liquid-phase process. The specific steps were shown hereinafter.

A 250 mL high-pressure reaction kettle made of Inconel alloy was used as a second reactor. 20 g of 3-chloro-1,1,1,2-tetrafluoropropane (having a purity of 99.8%), 40 g of KOH solution (25wt%), and 1 g of tetramethylammonium hydroxide were added in the reaction kettle. The reaction kettle was sealed, and 1.0 MPa nitrogen was introduced into the reaction kettle to replace the air in the reaction kettle. The replacement process repeated for three times. After the replacement process, the reaction temperature was set as 50 degrees centigrade, and the reaction performed under conditions of stirring at 300 rpm for 5 hours.

After the reaction, the reaction product was subjected to liquid separation and rectification to obtain the target product 2,3,3,3-tetrafluoropropene. The reaction product was analyzed by gas-phase chromatographic analysis. A conversion rate of 3-chloro-1,1,1,2-tetrafluoropropane was 90.9%, and a selectivity of the product 2,3,3,3-tetrafluoropropene was 96.7%.

### Embodiment 74

Method of the present embodiment was substantially the same as that in Embodiment 73, except that in the dehydrochlorination process, the KOH solution (25 wt%) was replaced with 40 g of NaOH solution (25 wt%). The other operations were not changed.

A product of the dehydrochlorination process was analyzed by online GC analysis and GC/MS analysis, a conversion rate of the 3-chloro-1,1,1,2-tetrafluoropropane was 88.8%, and a selectivity of the product 2,3,3,3-tetrafluoropropene was 97.5%.

### Embodiment 75

Method of the present embodiment was substantially the same as that in Embodiment 73, except that in the dehydrochlorination process, the reaction temperature increased to 80 degrees centigrade. The other operations were not changed.

A product of the dehydrochlorination process was analyzed by online GC analysis and GC/MS analysis, a conversion rate of the 3-chloro-1,1,1,2-tetrafluoropropane was 92.4%, and a selectivity of the product 2,3,3,3-tetrafluoropropene was 91.8%.

### Embodiment 76

Method of the present embodiment was substantially the same as that in Embodiment 57, except that the coconut shell activated carbon was replaced by coal-based activated carbon having a size in a range of 10 meshes to 20 meshes. The other operations were not changed.

A product of the dehydrochlorination process was analyzed by online GC analysis and GC/MS analysis, a conversion rate of the 2,3-dichloro-1,1,1,2-tetrafluoropropane was 90.8%, and a selectivity of the product 1-chloro-2,3,3,3-tetrafluoropropene was 95.1%.

### Embodiment 77

Method of the present embodiment was substantially the same as that in Embodiment 57, except that in the dehydrochlorination process, the reaction temperature was lowered to 300 degrees centigrade. The other operations were not changed.

A product of the dehydrochlorination process was analyzed by online GC analysis and GC/MS analysis, a conversion rate of the 2,3-dichloro-1,1,1,2-tetrafluoropropane was 67.8%, and a selectivity of the product 1-chloro-2,3,3,3-tetrafluoropropene was 97.9%.

### Embodiment 78

Method of the present embodiment was substantially the same as that in Embodiment 57, except that in the dehydrochlorination process, the reaction temperature was lowered to 320 degrees centigrade. The other operations were not changed.

A product of the dehydrochlorination process was analyzed by online GC analysis and GC/MS analysis, a conversion rate of the 2,3-dichloro-1,1,1,2-tetrafluoropropane was 80.4%, and a selectivity of the product 1-chloro-2,3,3,3-tetrafluoropropene was 96.7%.

### Embodiment 79

Method of the present embodiment was substantially the same as that in Embodiment 57, except that the dehydrochlorination process was performed by a liquid-phase process. The specific steps were shown hereinafter.

A 250 mL high-pressure reaction kettle made of Inconel alloy was used as a second reactor. 20 g of 2,3-dichloro-1,1,1,2-tetrafluoropropane (having a purity of 99.5%), 40 g of KOH solution (25wt%), and 1 g of tetramethylammonium hydroxide were added in the reaction kettle. The reaction kettle was sealed, and 1.0 MPa nitrogen was introduced into the reaction kettle to replace the air in the reaction kettle. The replacement process repeated for three times. After the replacement process, the reaction temperature was set as 50 degrees centigrade, and the reaction performed under conditions of stirring at 300 rpm for 5 hours.

After the reaction, the reaction product was subjected to liquid separation and rectification to obtain the target product 1-chloro-2,3,3,3-tetrafluoropropene. The reaction product was analyzed by gas-phase chromatographic analysis. A conversion rate of 2,3-dichloro-1,1,1,2-tetrafluoropropane was 84.1%, and a selectivity of the product 1-chloro-2,3,3,3-tetrafluoropropene was 97.8%

### Embodiment 80

Method of the present embodiment was substantially the same as that in Embodiment 79, except that in the dehydrochlorination process, the KOH solution (25 wt%) was replaced by 40 g of NaOH solution (25 wt%). The other operations were not changed.

A product of the dehydrochlorination process was analyzed by online GC analysis and GC/MS analysis, a conversion rate of the 2,3-dichloro-1,1,1,2-tetrafluoropropane was 81.0%, and a selectivity of the product 1-chloro-2,3,3,3-tetrafluoropropene was 97.3%.

### Embodiment 81

Method of the present embodiment was substantially the same as that in Embodiment 79, except that in the dehydrochlorination process, the reaction temperature increased to 80 degrees centigrade. The other operations were not changed.

A product of the dehydrochlorination process was analyzed by online GC analysis and GC/MS analysis, a conversion rate of the 2,3-dichloro-1,1,1,2-tetrafluoropropane was 89.9%, and a selectivity of the product 1-chloro-2,3,3,3-tetrafluoropropene was 90.9%.

## Claims

1. A method for preparing fluorochloropropane by an addition reaction, **characterized by** comprising
subjecting dichloromethane, difluoromethane, and a fluorine-containing olefin of Formula I to a liquid-phase addition reaction under the action of an addition catalyst, so as to prepare fluorochloropropane of Formula II, and a reaction equation is as follows: 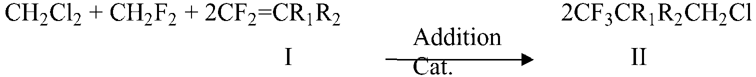
wherein R₁ is selected from hydrogen or fluorine, R₂ is selected from hydrogen or chlorine; and the addition catalyst is a mixed catalyst comprising a metal fluoride, a metal chloride and a metal oxide, and
a metal element of the metal fluoride is selected from metal elements in group IIIA, group IVB or group VA, a metal element of the metal chloride is selected from metal elements in group IIIA, group IVB or group VA, and a metal element of the metal oxide is selected from metal elements in group IIIA, group IVB or group VA.

2. The method of claim 1, wherein the metal is selected from the group consisting of Al, Sb, Ti, Zr, Hf, Nb, Ta, Ga, In, and any combination thereof.

3. The method of claim 2, wherein
the metal fluoride is selected from the group consisting of AlF₃, TiF₄, SbF₅, ZrF₄, HfF₄, and any combination thereof;
the metal chloride is selected from the group consisting of AlCl₃, TiCl₄, SbCl₅, ZrCl₄, HfCl₄, and any combination thereof; and
the metal oxide is selected from the group consisting of Al₂O₃, TiO₂, Sb₂O₅, ZrO₂, HfO₂, and any combination thereof.

4. The method of any one of claims 1 to 3, wherein the metal element of the metal fluoride, the metal element of the metal chloride and the metal element of the metal oxide are the same.

5. The method of claim 4, wherein in the addition catalyst, a molar content of fluorine element is in a range of 40% to 70% of a total anion molar content, a molar content of chlorine element is in a range of 20% to 50% of the total anion molar content, and a molar content of oxygen element is in a range of 10% to 30% of the total anion molar content.

6. The method of claim 5, wherein in the addition catalyst, the molar content of fluorine element is in a range of 50% to 60% of the total anion molar content, the molar content of chlorine element is in a range of 30% to 40% of the total anion molar content, and the molar content of oxygen element is in a range of 10% to 20% of the total anion molar content.

7. The method of any one of claims 1 to 6, further comprising monitoring a content of the fluorine-containing olefin in a reaction system during a reaction process, and controlling the content of the fluorine-containing olefin to be equal to or less than 5% of a mass of the reaction system.

8. The method of claim 1, wherein the fluorine-containing olefin is selected from the group consisting of vinylidene fluoride, chlorodifluoroethylene, trifluoroethylene, chlorotrifluoroethylene, and any combination thereof, and the fluorochloropropane is selected from the group consisting of 1-chloro-3,3,3-trifluoropropane, 2,3-dichloro-1,1,1-trifluoropropane, 3-chloro-1,1,1,2-tetrafluoropropane, 2,3-dichloro-1,1,1,2-tetrafluoropropane, and any combination thereof.

9. The method of claim 1, wherein a molar ratio of the dichloromethane to the fluorine-containing olefin is in a range of 0.5 : 1 to 2 : 1.

10. The method of claim 1, wherein a weight ratio of the dichloromethane to the addition catalyst is in a range of 2 to 10.

11. The method of claim 1, wherein in the addition reaction, a total amount of the fluorine-containing olefin satisfies a following relationship: ${n}_{{CF}_{2} = {CR}_{1} {R}_{2}} \leq \frac{1}{2} {n}_{{CH}_{2} {Cl}_{2}}$, and ${n}_{{CF}_{2} = {CR}_{1} {R}_{2}} \leq \frac{1}{2} {n}_{{CH}_{2} {F}_{2}}$ wherein *n*_{*CF*₂=*CR*₁*R*₂} represents a molar amount of the fluorine-containing olefin, *n*_{*CH*₂*F*₂} represents a molar amount of the difluoromethane, and *n*_{*CH*₂*Cl*₂} represents a molar amount of the dichloromethane.

12. The method of claim 1, wherein in the addition reaction, the fluorine-containing olefin is fed under pressure via a metering pump.

13. The method of claim 12, wherein in the addition reaction, when a content of the fluorine-containing olefin is monitored to be greater than 5%, reducing a feeding rate of the fluorine-containing olefin.

14. The method of claim 12, wherein a temperature of the addition reaction is in a range of -20 degrees centigrade to 100 degrees centigrade, and a duration of the addition reaction is in a range of 4 hours to 20 hours.

15. The method of claim 14, wherein the temperature of the addition reaction is in a range of 0 degrees centigrade to 80 degrees centigrade, and the duration of the addition reaction is in a range of 6 hours to 15 hours.

16. A method for preparing a fluorine-containing propylene, **characterized by** comprising
step (1), preparing a fluorochloropropane intermediate by the method of any one of claims 1 to 15; and
step (2), subjecting the fluorochloropropane intermediate to a dehydrochlorination reaction under the action of activated carbon or a metal alkali solution to obtain the fluorine-containing propylene.

17. The method of claim 16, wherein step (1) further comprises following steps:
preparing a fluorochloropropane liquid by the method of any one of claims 1 to 15,
subjecting the fluorochloropropane liquid to solid-liquid separation to remove the addition catalyst, and
subjecting the fluorochloropropane liquid after the solid-liquid separation to rectification to obtain the fluorochloropropane intermediate.

18. The method of claim 16, wherein in step (2), an activated carbon catalyst is selected from the group consisting of nut shell activated carbon, coal-based activated carbon, wood-based activated carbon, and any combination thereof.

19. The method of claim 18, wherein in step (2), a temperature of the dehydrochlorination reaction is in a range of 200 degrees centigrade to 500 degrees centigrade.

20. The method of claim 16, wherein in step (2), the metal alkali solution is selected from the group consisting of a sodium hydroxide solution, a potassium hydroxide solution, a sodium carbonate solution, a potassium carbonate solution, a sodium bicarbonate solution, a potassium bicarbonate solution, and any combination thereof, and a mass concentration of the metal alkali solution is in a range of 5 wt% to 60 wt%.

21. The method of claim 20, wherein in step (2), subjecting the fluorochloropropane intermediate to the dehydrochlorination reaction to obtain the fluorine-containing propylene under the action of the metal alkali solution and a quaternary ammonium salt, and the quaternary ammonium salt is selected from the group consisting of tetrabutylammonium bromide, tetramethylammonium hydroxide, tetramethylammonium chloride, and any combination thereof.

22. The method of claim 20 or 21, wherein in step (2), a molar ratio of the fluorochloropropane intermediate to a metal alkali is in a range of 1 : 0.1 to 1: 5, and a temperature of the dehydrochlorination reaction is in a range of 10 degrees centigrade to 100 degrees centigrade.
